# DEMANDE DE BREVET EUROPEEN

(11) **EP 1 844 760 A1**
(43) Date de publication de la demande: **17.10.2007**
(21) Numéro de dépôt: 07106003.2
(22) Date de dépôt: 12.04.2007
(51) Int. Cl.: A61K 8/81, A61K 8/40, A61K 8/36, A61K 8/19, A61K 8/97, A61Q 5/00

(54) **Utilisation d'une composition cosmétique comprenant au moins un monomère cyanoacrylate polymérisable et au moins un agent de conditionnement et/ou au moins un composé additionnel particulier pour améliorer la couleur de fibres kératiniques teintes artificiellement.**

(30) Priorité: 13.04.2006 FR 0603279
(71) Demandeur: L'Oréal, 75008 Paris (FR)
(72) Inventeur: Dorkel, Jocelyne, 95130, LE PLESSIS-BOUCHARD (FR); Vic, Gabin, 60280, VENETTE (FR); Samain, Henri, 91570, BIEVRES (FR)
(74) Mandataire: Casalonga, Axel

(57) **Abrégé**

L'invention a pour objet l'utilisation d'une composition cosmétique comprenant au moins un monomère cyanoacrylate polymérisable et au moins un agent de conditionnement et/ou au moins un composé additionnel choisi parmi les charges, les bases minérales ou organiques et les alcools inférieurs en C₁-C₈ pour améliorer la couleur de fibres kératiniques artificiellement teintes, notamment les fibres kératiniques humaines et plus particulièrement les cheveux humains, plus particulièrement pour protéger la couleur des fibres kératiniques teintes artificiellement ainsi que leurs propriétés cosmétiques vis-à-vis du lavage ou pour uniformiser la couleur de fibres kératiniques, notamment les fibres kératiniques humaines et plus particulièrement les cheveux humains suite à leur coloration.

L'invention porte également sur les procédés de traitement et de coloration ainsi que sur les agents de coloration multi-composants.

## Description

L'invention a pour objet l'utilisation d'une composition cosmétique comprenant au moins un monomère cyanoacrylate polymérisable et au moins un agent de conditionnement et/ou au moins un composé additionnel particulier, dans le but d'améliorer la coloration de fibres kératiniques artificiellement teintes, notamment les fibres kératiniques humaines et plus particulièrement les cheveux humains.

L'invention a également pour objet des procédés de pré- ou post-traitement de la coloration desdites fibres et des procédés de coloration.

Il est connu de teindre les fibres kératiniques notamment humaines et en particulier les cheveux avec des compositions tinctoriales contenant des précurseurs de colorant d'oxydation, appelés généralement bases d'oxydation. Ces bases d'oxydation sont des composés incolores ou faiblement colorés qui, associés à des produits oxydants, donnent naissance par un processus de condensation oxydative à des composés colorés. On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser poser, puis à rincer les fibres.

Les colorations qui en résultent sont des colorations particulièrement chromatiques qui sont cependant temporaires ou semi-permanentes car la nature des interactions qui lient les colorants directs à la fibre kératinique et leur désorption de la surface et/ou du coeur de la fibre sont responsables de leur faible puissance tinctoriale et de leur mauvaise tenue aux lavages.

La couleur artificielle des cheveux apportée par un traitement de coloration directe ou d'oxydation est généralement satisfaisante le jour de l'application. Toutefois, ces couleurs s'estompent progressivement du fait des lavages répétés, les reflets s'affadissent pour conduire à des couleurs moins vives et moins esthétiques. Ceci est particulièrement remarquable pour les teintes dites « chaudes » contenant des nuances de rouge ou cuivrées.

Jusqu'à présent, l'utilisation de produit-soins rincés et non rincés commercialisés n'améliore pas suffisamment la tenue de la couleur artificielle des cheveux.

Il est donc également nécessaire de mettre au point des moyens permettant de protéger la couleur artificielle de l'effet des lavages répétés et de l'effet du temps.

D'autre part, selon les traitements que les cheveux ont subis (déformation permanente ou décolorations et colorations successives), leur état peut être relativement hétérogène dans une chevelure. Lorsque l'on applique une coloration sur ces cheveux communément appelés cheveux sensibilisés, la couleur globale obtenue n'est pas toujours régulière. Il est donc intéressant de disposer de traitements qui appliqués avant une coloration permettent d'obtenir un rendu global plus uniforme, aussi appelé unisson.

Il serait donc intéressant de disposer de compositions permettant d'amoindrir l'effet inhérent de la qualité de la fibre kératinique à colorer sur la coloration capillaire attendue.

Il est connu de la demande de brevet FR 2 833 489 d'utiliser des compositions de traitement des cheveux comprenant des monomères électrophiles permettant d'obtenir un gainage des cheveux rémanent aux shampooings.

La Demanderesse vient de découvrir qu'il était possible d'utiliser des monomères électrophiles spécifiques en présence d'agents de conditionnement et/ou de composés particuliers dans le but de résoudre les problèmes mentionnés ci-dessus.

La demanderesse a plus particulièrement constaté qu'en appliquant sur la chevelure une telle composition, il se formait in situ un revêtement ou un gainage rémanent. Sans que cette explication soit limitative, il semble que ce soit les ions hydroxyde (OH⁻) contenus dans l'eau à pH neutre absorbée par les cheveux qui enclenchent le processus de polymérisation anionique à l'interface composition de traitement-cheveux. Les agents de conditionnement et/ou les composés additionnels particuliers présents dans cette composition se trouvent ainsi imbriqués dans la structure polymérique obtenue suite à la polymérisation in situ du monomère cyanoacrylate.

En effet, la réalisation d'un gainage particulier couvrant la fibre kératinique artificiellement colorée peut avoir un « effet barrière » évitant ainsi le départ des colorants au cours des shampooings successifs, préservant ainsi la couleur de la fibre.

De plus, la Demanderesse a constaté que en plus des effets obtenus sur la qualité et la rémanence des colorations capillaires, les propriétés cosmétiques, telles que le toucher agréable, la douceur ou la facilité de démêlage étaient également conservées au cours des lavages successifs.

De même, la réalisation d'un gainage particulier couvrant une fibre kératinique sensibilisée peut permettre d'uniformiser l'état de surface du cheveu avant la coloration, sans toutefois s'opposer à la montée des colorants, et ceci de manière durable.

La Demanderesse a également constaté que l'application de la composition cosmétique avant ou après une coloration capillaire permettait d'obtenir des dépôts très cosmétiques sur les cheveux, les cheveux n'étant pas collés entre eux et le dépôt étant lisse et pouvant être rémanent aux shampooings.

Selon la formulation choisie, ce dépôt peut s'accompagner d'un gain de masse et de propriétés coiffantes pour l'ensemble de la chevelure, ou au contraire avoir un toucher naturel non « visible ».

On entend par « fibres kératiniques humaines » par les cheveux, les poils notamment de barbe ou moustache, les cils, les sourcils.

On entend par « fibres kératiniques teintes artificiellement » par fibres kératiniques teintes par un procédé de coloration directe ou par un procédé de coloration d'oxydation.

On entend par « lavage », par une ou plusieurs applications sur les fibres kératiniques d'une composition aqueuse rincée, le plus souvent détergente telle qu'un shampooing. Cette expression inclut également les baignades en particulier en mer et en piscine.

Dans le cadre de la présente invention, on entend par « agent de conditionnement » tout agent ayant pour fonction l'amélioration des propriétés cosmétiques des cheveux, en particulier la douceur, le démêlage, le toucher, le lissage, l'électricité statique.

L'invention a donc pour objet l'utilisation d'une composition cosmétique comprenant au moins un monomère cyanoacrylate polymérisable et au moins un agent de conditionnement et/ou au moins un composé additionnel choisi parmi les charges, les bases minérales ou organiques et les alcools inférieurs en C₁-C₈, pour améliorer la couleur de fibres kératiniques artificiellement teintes, notamment les fibres kératiniques humaines et plus particulièrement les cheveux humains.

L'invention a aussi pour objet l'utilisation de ladite composition cosmétique pour protéger la couleur des fibres kératiniques teintes artificiellement ainsi que leurs propriétés cosmétiques vis-à-vis du lavage.

L'invention porte également sur l'utilisation de ladite composition cosmétique dans le but d'uniformiser la couleur de fibres kératiniques, notamment les fibres kératiniques humaines et plus particulièrement les cheveux humains suite à leur coloration.

L'invention porte également sur un procédé pour protéger la couleur des fibres kératiniques teintes artificiellement ainsi que leurs propriétés cosmétiques vis-à-vis du lavage.

L'invention a également pour objet un procédé de traitement des fibres kératiniques pour uniformiser la couleur desdites fibres suite à leur coloration,

L'invention a pour autre objet un procédé de coloration consistant à appliquer sur les fibres kératiniques humaines et plus particulièrement des cheveux, une composition (A) colorante, et de faire suivre ou précéder cette application par l'application d'une composition (B) contenant la composition cosmétique telle que définie précédemment.

L'invention a pour dernier objet des agents de coloration multi-composants.

Les différents objets de l'invention vont maintenant être détaillés. L'ensemble des significations et définitions des composés utilisés dans la présente invention données ci-dessous sont valables pour l'ensemble des objets de l'invention.

Le ou les monomères cyanoacrylate présents dans la composition de l'invention sont de préférence choisis parmi les monomères de formule (I) : 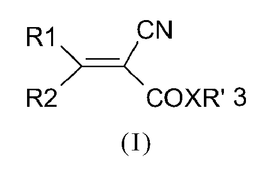 dans laquelle :
- X désigne NH, S ou O.
- R1 et R2 désignent chacun, indépendamment l'un de l'autre, un groupe peu ou non électro-attracteur (peu ou non inductif-attracteur) tel que :
- un atome d'hydrogène,
- un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
- un résidu polyorganosiloxane modifié ou non,
- un groupement polyoxyalkylène.
- R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', - COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀ ;
- R'₃ représentant un atome d'hydrogène ou un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀.

Par groupement électro-attracteur ou inductif-attracteur (-I), on entend tout groupement plus électronégatif que le carbone. On pourra se reporter à l'ouvrage PR Wells Prog. Phys. Org. Chem., Vol 6,111 (1968).

Par groupement peu ou non électro-attracteur, on entend tout groupement dont l'électronégativité est inférieure ou égale à celle du carbone.

Les groupements alcényle ou alcynyle ont de préférence 2 à 20 atomes de carbone, mieux encore de 2 à 10 atomes de carbone.

Comme groupe hydrocarboné saturé ou non, linéaire, ramifié ou cyclique, comportant de préférence de 1 à 20 atomes de carbone, on peut notamment citer les groupes alkyle, alcényle ou alcynyle linéaires ou ramifiés, tels que méthyle, éthyle, n-butyle, tert-butyle, iso-butyle, pentyle, hexyle, octyle, butényle ou butynyle ; les groupes cycloalkyle ou aromatiques.

Comme groupe hydrocarboné substitué, on peut citer par exemple les groupes hydroxyalkyle ou polyhalogénoalkyle.

A titre d'exemples de polyorganosiloxane non modifié, on peut notamment citer les polyalkylsiloxanes tels que les polydiméthylsiloxanes, les polyarylsiloxanes tels que les polyphénylsiloxanes, les polyarylalkylsiloxanes tels que les polyméthylphénylsiloxanes.

Parmi les polyorganosiloxanes modifiés, on peut notamment citer les polydiméthylsiloxanes à groupements polyoxyalkylène et / ou siloxy et/ou silanol et / ou amine et / ou imine et / ou fluoroalkyle.

Parmi les groupements polyoxyalkylène, on peut notamment citer les groupements polyoxyéthylène et les groupements polyoxypropylène ayant de préférence 1 à 200 motifs oxyalkylénés.

Parmi les groupements mono- ou polyfluoroalkyle, on peut notamment citer des groupements tels que -(CH₂)n-(CF₂)m-CF₃ ou -(CH₂)ₙ-(CF₂)ₘ-CHF₂ avec n = 1 à 20 et m = 1 à 20.

Les substituants R₁ et R₂ peuvent éventuellement être substitués par un groupement ayant une activité cosmétique. Les activités cosmétiques particulièrement utilisées sont obtenues à partir de groupements à fonctions colorantes, antioxydantes, filtres UV et conditionnantes.

A titre d'exemples de groupement à fonction colorante, on peut notamment citer les groupements azoiques, quinoniques, méthiniques, cyanométhiniques et triarylméthane.

A titre d'exemples de groupement à fonction antioxydante, on peut notamment citer les groupements de type butylhydroxyanisole (BHA), butylhydroxytoluène (BHT) ou vitamine E.

A titre d'exemples de groupement à fonction filtre UV, on peut notamment citer les groupements de types benzophénones, cinnamates, benzoates, benzylidène-camphres et dibenzoylméthanes.

A titre d'exemples de groupement à fonction conditionnante, on peut notamment citer les groupements cationiques et de type esters gras.

De préférence, R₁ et R₂ représentent un atome d'hydrogène.

De préférence, R'₃ est un groupe hydrocarboné saturé comportant de 1 à 10 atomes de carbone ou un alcènyle comportant 2 à 10 atomes de carbone.

De préférence, X désigne O.

A titre de composés de formule (I), on peut citer les monomères :
a) appartenant à la famille des 2-cyanoacrylate de polyfluoroalkyle tels que :
   l'ester 2,2,3,3-tétrafluoropropylique de l'acide 2-cyano-2-propénoïque de formule : 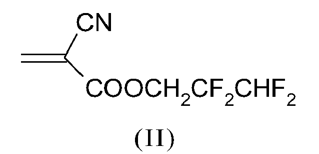
   ou encore l'ester 2,2,2-trifluoroéthylique de l'acide 2-cyano-2-propénoïque de formule : 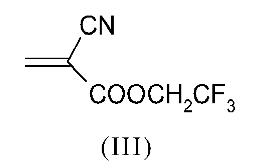
b) les 2-cyanoacrylate d'alkyle ou d'alcoxyalkyle de formule (IV) : 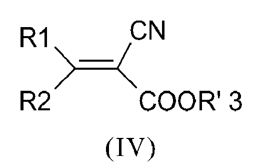 dans laquelle R'3 représente un radical alkyle en C₁-C₁₀ ou alcoxy(C₁-C₄) alkyle(C₁-C₁₀).

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate d'allyle, le 2-cyanoacrylate de 2-octyle, le 2-cyanoacrylate de 2-méthoxypropyle, le 2-cyanoacrylate de 2-propoxyéthyle, le 2-cyanoacrylate de n-octyle et le cyanoacrylate d'iso-amyle.

Dans le cadre de l'invention, on préfère utiliser les monomères b). Selon un mode de réalisation préféré, le ou les monomères cyanoacrylates sont choisis parmi les cyanoacrylates d'alkyle en C₆-C₁₀.

Les monomères particulièrement préférés sont les cyanoacrylate d'octyle de formule (V) et leurs mélanges : 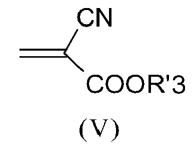 dans laquelle :
R'3 =-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Un monomère cyanoacrylate particulièrement préféré utilisé dans la composition cosmétique selon la présente invention est le méthylheptyl cyanoacrylate.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Le ou les monomères cyanoacrylate polymérisable est ou sont présents dans une quantité allant de préférence de 0,1 à 99 % en poids, de préférence dans une quantité allant de 1 à 30 % en poids par rapport au poids total de la composition cosmétique.

Les monomères cyanoacrylate de formule (I) selon la présente invention peuvent être synthétisés selon les méthodes connues décrites dans la technique. En particulier, les monomères cyanoacrylate peuvent être synthétisés selon l'enseignement des documents US 3 527 224, US 3 591 767, US 3 667 472, US 3 995 641, US 4 035 334 et US 4 650 826.

Les agents de conditionnement peuvent se présenter sous forme liquide, semi-solide ou solide tels que par exemple des huiles, des cires ou des gommes.

Les agents de conditionnement selon l'invention sont choisis de préférence parmi :
- les acides organiques,
- les huiles

L'agent de conditionnement selon l'invention peut être un acide organique, de préférence en C₁-C₁₂, ce dernier ayant un ou plusieurs groupements carboxyliques ou sulfoniques, présentant un pKa compris entre 0 et 6 tels que l'acide benzène- ou toluène-sulfonique, l'acide acétique, l'acide formique, l'acide propionique, l'acide benzoïque, les acides mono-, di- ou trichloroacétiques, l'acide salicylique et l'acide trifluoroacétique, l'acide citrique, l'acide octanoïque, l'acide heptanoïque et l'acide hexanoïque.

De préférence, l'acide acétique, citrique ou octanoïque est utilisé.

Préférentiellement, l'acide organique est introduit dans la composition entre 0,01% et 30% en poids par rapport au poids total de la composition ; plus préférentiellement entre 0,1% et 15% en poids par rapport au poids total de la composition.

L'agent de conditionnement selon l'invention peut également être une huile, telle que les huiles minérales, organiques ou végétales.

De préférence, l'huile peut être une huile végétale, de préférence un triglycéride.

On citera en particulier : l'huile d'olive, l'huile de ricin, l'huile de colza, l'huile de coprah, l'huile de germes de blé, l'huile d'amandes douces, l'huile d'avocat, l'huile de polybutène, l'huile de macadamia, l'huile d'amandes d'abricot, l'isononate d'isononyle, l'huile de carthame, l'huile de noix de bancoulier, le malate d'isostéaryle, l'huile de jojoba, l'huile de tournesol, l'huile de sésame, l'huile d'arachide, l'huile de pépins de raisin, l'huile de soja, e, l'huile de coprah, l'huile de maïs, l'huile de noisette, le beurre de karité, l'huile de palme, l'huile de calophyllum, l'huile de camelina, l'huile d'amandes tamanu, le tetra-isostéarate de pentaérythrityle, le trimellitate de tridécyle, l'huile de citron.

Selon l'invention, l'huile peut également être une silicone volatile. Les silicones volatiles utilisables dans l'invention sont des silicones linéaires ou cycliques, ayant une viscosité à température ambiante et sous pression atmosphérique, inférieure à 8 mm2/s (8 cSt).

La viscosité est mesurée de préférence par viscosimétrie capillaire, par exemple, à l'aide d'un viscosimètre capillaire, notamment de type Ubbelohde, à une température de 25 °C, selon la norme ASTM D445-97. On peut aussi utiliser la méthode dite de la chute de la bille.

Les silicones volatiles présentent généralement un point d'ébullition compris entre 60 C et 260° C, et sont plus particulièrement choisies parmi :
(i) les silicones volatiles cycliques comportant de 3 à 7 atomes de silicium et, de préférence, 4 à 5. Il s'agit, par exemple, de l'octaméthylcyclotétrasiloxane commercialisé notamment sous le nom de "VOLATILE SILICONE 7207" par UNION CARBIDE ou "SILBIONE^{®} 70045 V 2" par RHODIA, le décaméthylcyclopentasiloxane commercialisé sous le nom de "VOLATILE SILICONE 7158" par UNION CARBIDE, "SILBIONE^{®} 70045 V 5" par RHODIA ou sous le nom DC245 Fluid par DOW CORNING, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique :
   avec 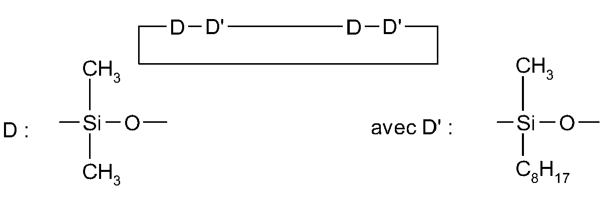 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5 mm²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

De préférence, le décaméthylcyclopentasiloxane appelé plus communément D5 est utilisé.

Selon l'invention, l'huile peut également être une silicone fluide.

Par silicone fluide, on entend une silicone dont la viscosité dynamique mesurée à 25°C est comprise entre 0,1 est et 1 000 000 est, et de préférence entre lest et 30 000 est.

Les silicones utilisables conformément à l'invention peuvent être solubles ou insolubles dans l'eau, et elles peuvent être en particulier des polyorganosiloxanes insolubles dans l'eau ;

Les organopolysiloxanes sont définis plus en détail dans l'ouvrage de Walter NOLL "Chemistry and Technology of Silicones" (1968) Academic Press. Elles peuvent être volatiles ou non volatiles.

Parmi les silicones non volatiles fluides, on peut notamment citer les polyalkylsiloxanes, les polyarylsiloxanes, les polyalkylarylsiloxanes, les gommes de silicones, les polyorganosiloxanes modifiés par des groupements organofonctionnels.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C6-C24 tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C12)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C1-C4 ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732 ;
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL® S201" et "ABIL® S255" ;
- des groupements hydroxyacylamino, comme les polyorganosiloxanes décrits dans la demande EP 342 834. On peut citer, par exemple, le produit Q2-8413 de la société DOW CORNING.

De préférence, les huiles silicones dans les compositions selon l'invention constituées d'une huile volatile PDMS (PolyDiMéthylSiloxane) et d'une gomme diméthicone ou diméthiconol. Parmi les mélanges déjà commerciaux, on peut citer
- les mélanges D5 et diméthiconol comme la DC 1501 Fluid (Dow Corning),
- les mélanges D5 et diméthicone de type DC1411 Fluid de Dow Corning et SF1214 de Bayer,
- les mélanges PDMS fluide et diméthiconol de type DC1503 Fluid de Dow Corning.

Préférentiellement l'huile est introduite dans la composition entre 1% et 99.9% en poids par rapport au poids total de la composition, plus préférentiellement entre 20% et 95% en poids par rapport au poids total de la composition.

Lorsque le composé additionnel est choisi parmi les charges, lesdites charges peuvent être choisies parmi les charges minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, la silice traitée en surface par un agent hydrophobe, le kaolin, la bentone, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la Société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

On considère de préférence la silice pyrogénée éventuellement traitée hydrophobe en surface. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :
- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6ème édition, 1995). Elles sont par exemple commercialisées sous les références « Aerosil R812® » et» « Aerosil R8200 » par la société DEGUSSA, « CAB-O-SIL TS-530®» par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (6ème édition, 1995). Elles sont par exemple commercialisées sous les références « Aerosil R972® », et « Aerosil R974® « par la société DEGUSSA, « CAB-O-SIL TS-610® » et « CAB-O-SIL TS-720® » par la société CABOT.

La silice pyrogénée hydrophobe présente en particulier une taille de particules pouvant être nanométrique à micrométrique, par exemple allant d'environ de 5 à 200 nm.

On considère également de préférence le nitrure de bore comme la gamme Belsil BNP de Wacker ou le CC6098 ou CC6004 de Advanced Ceramics.

Les particules peuvent être traitées par enrobage ou greffage. On peut ainsi notamment obtenir des particules mixtes minéral/organique.

Les particules peuvent également être des composés qui ont été rendus hydrophiles par enrobage ou greffage chimique à l'aide de matières telles que le chitosane, le dioxyde de titane, la silice ou des polymères hydrophiles, notamment des polyesters sulfoniques ou des polyammonium quaternaire.

On peut également choisir des composés pulvérulents hydrophobes, choisis parmi des composés pulvérulents de nature aussi bien hydrophobe qu'hydrophile. Dans ce dernier cas, ils sont rendus hydrophobes par enrobage ou greffage chimique par des produits tels que les silicones, les aminoacides, les savons métalliques, les dérivés fluorés, les huiles minérales, la lécithine, le triisostéaroyl titanate d'isopropyle, le polyéthylène, le collagène et ses dérivés, les polyacrylates.

A titre d'exemple, on peut citer les microbilles de silice enrobées de polyméthylhydrogénosiloxane vendues sous la dénomination commerciale "Silice SI SB 700" par Miyoshi ou encore la sericite enrobée de methicone et d'huile d'oeuf hydrogénée vendue sous la dénomination commerciale "Sericite SNI S100" par Miyoshi.

Préférentiellement, les charges peuvent être présentes dans la composition à des teneurs comprises entre 0,01 et 50% en poids, de préférence entre 0,01% et 30%, en poids du poids total de la composition.

Lorsque le composé additionnel est une base minérale ou organique, ladite base est choisie parmi l'ammoniaque, la monoéthanolamine, la diéthanolamine, la triéthanolamine, la 1,3-propanediamine, un carbonate ou un bicarbonate alcalin ou d'ammonium, un carbonate organique tel que le carbonate de guanidine, un hydroxyde alcalin tel que la soude ; de préférence la monoéthanolamine.

Préférentiellement, la base est introduite dans la composition entre 0,01% et 30% en poids par rapport au poids total de la composition ; plus préférentiellement entre 0,01% et 5% en poids par rapport au poids total de la composition.

Lorsque le composé additionnel est un alcool inférieur en C₁-C₈ utilisé, l'alcool peut être choisi parmi les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, et de préférence l'éthanol.

Préférentiellement, l'alcool est introduit dans la composition entre 0,01% et 30% en poids par rapport au poids total de la composition ; plus préférentiellement entre 0,01% et 5% en poids par rapport au poids total de la composition.

Dans le cadre de la présente invention, les monomères électrophiles cyanoacrylate de formule (I) sont des monomères capables de polymériser par voie anionique en présence d'un agent nucléophile. Par polymérisation anionique, on entend le mécanisme défini dans l'ouvrage "Advanced Organic Chemistry", Third Edition de Jerry March, pages 151 à 161.

Les agents nucléophiles susceptibles d'initier la polymérisation anionique sont des systèmes connus en eux-mêmes, capables de générer un carbanion au contact d'un agent nucléophile, tels que les ions hydroxydes contenus dans l'eau à pH neutre. On entend par " carbanion ", les espèces chimiques définies dans " Advanced Organic Chemistry, Third Edition ", de Jerry March, page 141.

Les agents nucléophiles peuvent être appliqués séparément de la composition de l'invention. Ils peuvent être mélangés à la composition de l'invention au moment de l'emploi.

L'agent nucléophile est un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PhNH⁻, pyridine, ArS⁻, R-C^{≡}C⁻,

RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻, H₂O, Ph représentant un groupe phényle ; Ar représentant un groupe aryle et R représentant un groupe alkyle en C₁-C₁₀.

On peut également introduire dans les compositions des inhibiteurs de polymérisation autres que des acides organiques et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, le trifluorure de bore, l'hydroquinone et ses dérivés tels que l'hydroquinone monoéthyléther, la TBHQ, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges. Les groupements alkyle désignent de préférence des groupement ayant 1 à 6 atomes de carbone.

La concentration en inhibiteur de polymérisation dans la composition cosmétique de l'invention peut être comprise entre 10 ppm et 5%, et plus préférentiellement entre 10 ppm et 0,5% en poids par rapport au poids total de la composition.

La composition mise en oeuvre dans l'utilisation selon l'invention peut comprendre des filtres UV organiques (systèmes filtrant les radiations UV) choisis parmi les filtres hydrosolubles ou liposolubles, siliconés ou non siliconés.

Les filtres organiques sont notamment choisis parmi les dérivés de dibenzoylméthane ; les anthranilates ; les dérivés cinnamiques ; les dérivés salicyliques, les dérivés du camphre ; les dérivés de la benzophénone ; les dérivés de ,-diphénylacrylate ; les dérivés de triazine ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres UV organiques, on peut citer ceux désignés ci-dessous sous leur nom INCI :
Dérivés de l'acide para-aminobenzoïque :
   PABA,
   Ethyl PABA,
   Ethyl Dihydroxypropyl PABA,
   Ethylhexyl Diméthyl PABA vendu notamment sous le nom « ESCALOL 507 » par ISP,
   Glyceryl PABA,
   PEG-25 PABA vendu sous le nom « UVINUL P25 » par BASF,
Dérivés cinnamiques :
   Ethylhexyl Methoxycinnamate vendu notamment sous le nom commercial « PARSOL MCX » par HOFFMANN LA ROCHE,
   Isopropyl Methoxy cinnamate,
   Isoamyl Methoxy cinnamate vendu sous le nom commercial « NEO HELIOPAN E 1000 » par HAARMANN et REIMER,
   Cinoxate,
   DEA Methoxycinnamate,
   - Diisopropyl Methylcinnamate,
   Glyceryl Ethylhexanoate Dimethoxycinnamate
Dérivés du dibenzoylméthane :
   Butyl Methoxydibenzoylmethane vendu notamment sous le nom commercial « PARSOL 1789 » par HOFFMANN LA ROCHE,
   Isopropyl Dibenzoylmethane vendu notamment sous le nom commercial « EUSOLEX 8020 » par MERCK,
Dérivés salicyliques :
   Homosalate vendu sous le nom « Eusolex HMS » par Rona/EM Industries,
   Ethylhexyl Salicylate vendu sous le nom « NEO HELIOPAN OS » par Haarmann et REIMER,
   Dipropyleneglycol Salicylate vendu sous le nom « DIPSAL » par SCHER,
   TEA Salicylate, vendu sous le nom « NEO HELIOPAN TS » par Haarmann et REIMER,
Dérivés de diphénylacrylate :
   Octocrylene vendu notamment sous le nom commercial « UVINUL N539 » par BASF,
   Etocrylene, vendu notamment sous le nom commercial « UVINUL N35 » par BASF,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF,
   Benzophenone-5
   Benzophenone-6 vendu sous le nom commercial « Helisorb 11 » par Norquay
   Benzophenone-8 vendu sous le nom commercial « Spectra-Sorb UV-24 » par American Cyanamid
   Benzophenone-9 vendu sous le nom commercial« UVINUL DS-49» par BASF,
   Benzophenone-12
   2-(4-diethylamino-2-hydroxybenzoyl)-benzoate de n-hexyle.
Dérivés du benzylidène camphre :
   3-Benzylidene camphor fabriqué sous le nom « MEXORYL SD» par CHIMEX,
   4-Methylbenzylidene camphor vendu sous le nom « EUSOLEX 6300 » par MERCK,
   Benzylidene Camphor Sulfonic Acid fabriqué sous le nom « MEXORYL SL» par CHIMEX,
   Camphor Benzalkonium Methosulfate fabriqué sous le nom « MEXORYL SO » par CHIMEX,
   Terephthalylidene Dicamphor Sulfonic Acid fabriqué sous le nom « MEXORYL SX » par CHIMEX,
   Polyacrylamidomethyl Benzylidene Camphor fabriqué sous le nom « MEXORYL SW » par CHIMEX,
Dérivés du phényl benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate vendu sous le nom commercial commercial « NEO HELIOPAN AP » par Haarmann et REIMER,
Dérivés du phényl benzotriazole :
   Drometrizole Trisiloxane vendu sous le nom « Silatrizole » par RHODIA CHIMIE ,
   Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés de triazine :
   Bis-Ethylhexyloxyphenol Methoxyphenyl Triazine vendu sous le nom commercial «TINOSORB S » par CIBA GEIGY,
   Ethylhexyl triazone vendu notamment sous le nom commercial «UVINUL T150 » par BASF,
   Diethylhexyl Butamido Triazone vendu sous le nom commercial « UVASORB HEB » par SIGMA 3V,
   la 2,4,6-tris-(4'-amino benzalmalonate de diisobutyle)-s-triazine.
Dérivés anthraniliques :
   Menthyl anthranilate vendu sous le nom commercial commercial « NEO HELIOPAN MA » par Haarmann et REIMER,
Dérivés d'imidazolines :
   Ethylhexyl Dimethoxybenzylidene Dioxoimidazoline Propionate,
Dérivés du benzalmalonate :
   Polyorganosiloxane à fonctions benzalmalonate comme le Polysilicone-15 vendu sous la dénomination commerciale « PARSOL SLX » par HOFFMANN LA ROCHE
Dérivés de 4,4-diarylbutadiène :
   -1,1-dicarboxy (2,2'-diméthyl-propyl)-4,4-diphénylbutadiène
Dérivés de benzoxazole :
   2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V
   et leurs mélanges.

Comme filtres UV organiques liposolubles (ou lipophiles) convenant à une mise en oeuvre dans la présente invention, on peut citer plus particulièrement :
Ethylhexyl Methoxycinnamate
Butyl Methoxydibenzoylmethane
Homosalate
Ethylhexyl Salicylate,
Octocrylene,
Benzophenone-3,
2-(4-diethylamino-2-hydroxybenzoyl)-benzoate de n-hexyle.
4-Methylbenzylidene camphor,
Ethylhexyl triazone,
Bis-Ethylhexyloxyphenol Methoxyphenyl Triazine
Diethylhexyl Butamido Triazone,
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine

Comme filtres UV organiques hydrosolubles (ou hydrophiles) convenant à une mise en oeuvre dans la présente invention, on peut citer plus particulièrement:
PABA,
PEG-25 PABA
Benzylidene Camphor Sulfonic Acid,
Camphor Benzalkonium Methosulfate
Terephthalylidene Dicamphor Sulfonic Acid
Phenylbenzimidazole Sulfonic Acid
Disodium Phenyl Dibenzimidazole Tetra-sulfonate
Benzophenone-4
Benzophenone-5.

La composition mise en oeuvre dans l'utilisation selon l'invention peut également comprendre des polymères.

Par « polymère », on entend tous les polymères utilisables en cosmétique, naturels ou synthétiques et en particulier les polymères obtenus par polymérisation radicalaire ou anionique ou par polycondensation ou par ouverture de cycles. Ces polymères peuvent être linaires, ramifiés ou en étoile.

La composition selon l'invention peut également comprendre des agents réducteurs choisis parmi les thioacides et leurs sels (acide thioglycolique ou thiosulfate, cystéine ou cystéamine), les sulfites de métaux alcalins ou alcalinoterreux, les sucres réducteurs tels que le glucose, la vitamine C et ses dérivés, les dérivés d'acide sulfovinique, les phosphines.

La composition selon l'invention peut également comprendre des agents colorants choisis parmi les structures conjuguées linéaires ou aromatiques (hétérocycliques ou non). Parmi celles-ci, on peut citer les colorants benzéniques nitrés, les colorants aromatiques, les colorants amino-benzéniques, les colorants azoïques, les colorants anthraquinoniques, les diamines aromatiques, les aminophénols, les phénols et naphtols, les porphyrines, tétraphénylporphyrines, métalloporphyrines, les phtalocyanines, les caroténoïdes, les flavonoïdes, les molécules fluorescentes (fluoresceine, rhodamine, coumarine .....)

Le milieu de la composition selon l'invention peut contenir de l'eau ou un ou plusieurs solvants organiques liquides, ou un mélange d'eau et d'un ou plusieurs solvants organiques liquides.

De manière préférentielle, le support de la composition selon l'invention sera de préférence anhydre et non hygroscopique.

Au sens de la présente invention, on entend par support anhydre, un support contenant moins de 1 % d'eau.

De préférence les solvants organiques liquides sont choisis parmi les composés liquides à la température de 25°C et sous 105Pa (760mm de Hg) et différents des monomères cyanoacrylate selon l'invention.

Le support peut être constitué notamment par le ou les agents de conditionnements tels que cités ci-dessus.

Parmi les solvants organiques liquides utilisables, on peut citer ceux choisis parmi :
- les alcools aromatiques tels que l'alcool benzylique ;
- les alcools gras liquides comportant au moins 12 atomes de carbone;
- les paraffines liquides, les alcanes liquides et plus particulièrement les alcanes de C5 à C10 ;
- les acides gras liquides comportant au moins 12 atomes de carbone, les amides grasses liquides, les esters gras et plus particulièrement les benzoates ou les salicylates d'alcool gras.

Selon une forme particulière de l'invention, le support de la composition de l'invention peut se présenter sous forme de capsules en dispersion, soit dans un milieu anhydre tel que défini précédemment, soit dans un milieu aqueux ou sous la forme d'une émulsion.

Selon une forme particulière de l'invention, le support de la composition de l'invention peut se présenter sous forme d'émulsion comprenant une phase aqueuse et une phase organique dans lequel le monomère cyanoacrylate est stabilisé.

La composition cosmétique de l'invention peut également contenir des additifs cosmétiques usuels choisis parmi cette liste non exhaustive tels que les agents réducteurs, les agents oxydants, les séquestrants, les agents épaississants polymériques ou non, les agents hydratants, les agents émollients, les plastifiants, les azurants optiques, les argiles, les minéraux colloïdaux, les métaux colloïdaux, les particules de semi-conducteurs de type «puits quantiques» à base de métaux ou de silicium, un composé photo ou thermochromique, les agents nacrant, les parfums, les peptisants, les conservateurs, les protéines, les vitamines, les agents antipelliculaires, les cires oxyéthylénées ou non, les polymères anioniques, cationiques ou amphotères fixants ou non, les tensioactifs anioniques, cationiques, amphotères et non ioniques.

Les formulations peuvent se présenter sous différentes formes galéniques tels qu'une lotion, une mousse aérosol, un après shampooing ou un shampooing, un gel, une cire. Les compositions peuvent être contenues dans un flacon pompe, un spray aérosol. Les compositions de l'invention après application sur la chevelure peuvent être rincées ou non.

Lorsque la composition est contenue dans un aérosol, elle peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non (butane, propane, isobutane) et leurs mélanges.

Selon un procédé de l'invention, la composition de l'invention est appliquée sur les fibres kératiniques, en particulier les fibres kératiniques humaines telles que les cheveux, en présence d'un agent nucléophile.

Selon un mode de réalisation particulier du procédé de l'invention, l'agent nucléophile susceptible d'initier la polymérisation du monomère cyanoacrylate peut être appliqué au préalable sur les fibres kératiniques. L'agent nucléophile peut être utilisé pur, en solution, sous forme d'une émulsion ou être encapsulé. Il peut aussi être ajouté à la composition anhydre au moment de l'emploi juste avant l'application sur les fibres kératiniques.

De préférence, cet agent nucléophile est l'eau. Cette eau peut être apportée par exemple par humidification préalable des fibres kératiniques. Elle peut aussi être ajoutée directement dans la composition avant application.

Selon un mode de réalisation particulier, il est possible de moduler la cinétique de polymérisation en humidifiant préalablement la fibre à l'aide d'une solution aqueuse dont le pH a été ajusté à l'aide d'une base, d'un acide ou d'un mélange acide/base. L'acide et/ou la base peuvent être inorganique ou organique.

Il est aussi possible de réaliser des applications multiples de la composition de l'invention afin d'obtenir une superposition de couches pour atteindre des propriétés spécifiques du dépôt en termes de nature chimique, résistance mécanique, épaisseur, aspect, toucher.

Afin d'améliorer entre autre l'adhésion du poly(cyanoacrylate) formé in situ, la fibre peut être prétraitée avec tous types de polymères.

Pour moduler la cinétique de polymérisation anionique, on peut également augmenter la nucléophilie de la fibre par transformation chimique des fibres kératiniques. A titre d'exemple, on peut citer la réduction des ponts di-sulfure composant en partie la kératine en thiols avant application de la composition de l'invention. De façon non exhaustive, on peut citer comme réducteurs des ponts di-sulfure composant en partie la kératine, les composés suivants: thiosulfate de sodium anhydre, métabisulfite de sodium en poudre, thiourée, sulfite d'ammonium, acide thioglycolique, acide thiolactique, thiolactate d'ammonium, mono-thioglycolate de glycérol, thioglycolate d'ammonium, thioglycérol, acide 2,5-dihydroxybenzoique, di-thioglycolate de diammonium, thioglycolate de strontium, thioglycolate de calcium, formo-sulfoxylate de zinc, thioglycolate d'isooctyle, dl-cystéine, thioglycolate de monoéthanolamine.

L'invention a également pour objet un procédé pour améliorer la couleur des fibres kératiniques teintes artificiellement, caractérisé par le fait que l'on applique avant ou après coloration desdites fibres, au moins une composition cosmétique telle que définie précédemment en présence d'au moins un agent nucléophile tel que défini ci-dessus; ledit agent nucléophile étant présent dans ladite composition ou conditionné séparément.

Selon une variante de l'invention, lorsque la composition selon l'invention est appliquée sur les fibres après la coloration capillaire, le procédé permet de protéger la couleur des lavages successifs et d'améliorer les propriétés cosmétiques suite à ces lavages.

Ce procédé peut se réaliser en un temps ou en deux temps. Lorsque le procédé de protection de la couleur se fait en deux temps, il comprend les étapes suivantes. Suite à une étape de coloration capillaire et à une étape d'essorage, une composition contenant un agent nucléophile est appliquée sur lesdites fibres. Les fibres sont ensuite éventuellement séchées. Puis dans un second temps, une composition cosmétique telle que définie ci-dessus et ne contenant pas l'agent nucléophile est appliquée sur lesdites fibres.

Il est possible de prévoir, préalablement au dépôt de la composition contenant un agent nucléophile, l'application sur les fibres d'une composition contenant un additif cosmétique

Il est aussi possible de prévoir, une étape de rinçage et/ou une étape de lavage au shampooing, avant l'application de la composition contenant le monomère cyanoacrylate polymérisable et l'agent de conditionnement et/ou le composé additionnel.

Les procédés selon l'invention peuvent comprendre une étape supplémentaire de séchage total ou partiel des fibres kératiniques avec un séchoir et éventuellement un shampooing terminal.

Selon une forme particulière de l'invention, le procédé de protection de la couleur des fibres kératiniques peut comprendre une étape de chauffage de la composition cosmétique que l'on appliquera ensuite directement sur les fibres kératiniques. La température sera de préférence de 60 à 120°C.

Selon une forme particulière de l'invention, le procédé de traitement de la couleur des fibres kératiniques peut comprendre une étape de chauffage des fibres kératiniques après application de la composition cosmétique.

Le chauffage des fibres kératiniques peut par exemple être effectué au moyen d'un fer, d'un mélange eau liquide/vapeur d'eau ou bien au moyen d'un casque chauffant.

Le fer chauffant utile dans le cadre de l'invention est un fer chauffant conventionnellement utilisé dans le domaine capillaire. Un tel fer, par exemple un fer à friser ou un fer à lisser, est bien connu dans le domaine du traitement capillaire. Par exemple, des fers utiles pour mettre en oeuvre la présente invention sont des fers plats ou ronds décrits dans les brevets US 4 103 145, US 4 308 878, US 5 983 903, US 5 957 140, US 5 494 058. L'application du fer peut se faire par touches séparées successives de quelques secondes, ou par déplacement ou glissement progressif le long des mèches. Il est possible entre l'application de la composition protectrice de la couleur et l'application du fer chauffant sur les fibres kératiniques de prévoir un temps de pause. Ledit temps de pause de préférence variera de 30 secondes à 60 minutes et plus préférentiellement de 1 à 30 minutes.

Le mélange eau liquide/vapeur d'eau utile dans le cadre de l'invention a en général une température d'au moins 35°C.

Le mélange eau liquide/vapeur d'eau constitue une brume. Ledit mélange peut contenir en plus au moins un autre gaz tel que l'oxygène ou l'azote, des mélanges de gaz tels que l'air ou bien encore d'autres composés vaporisables.

La température du mélange eau liquide/vapeur d'eau est de préférence supérieure ou égale à 40°C, et est plus particulièrement comprise entre 40°C et 75°C environ.

De préférence, le mélange eau liquide/vapeur d'eau est mis en contact avec la fibre pendant une durée allant de 1 seconde à 1 heure, et encore plus préférentiellement de 5 minutes à 15 minutes. Bien entendu, l'application dudit mélange peut être répétée plusieurs fois sur la même fibre, chaque opération se faisant selon une durée telle qu'indiquée ci-dessus. Plus préférentiellement, on applique tout d'abord sur les cheveux la composition cosmétique, puis on soumet ces mèches ainsi imprégnées à l'action du mélange eau liquide/vapeur d'eau selon les conditions mentionnées précédemment, puis on refroidit les mèches ainsi traitées par exemple en envoyant sur ou à travers celles-ci un courant d'air froid ou d'air à température ambiante.

La production du mélange eau liquide/vapeur d'eau utilisé selon l'invention peut se faire à l'aide de tout appareil connu en soi et prévu à cet effet. Toutefois, selon la présente invention, on utilise de préférence un appareil comprenant au moins un générateur de vapeur d'eau directement relié à un casque diffusant sur les fibres kératiniques en particulier les cheveux humains le mélange eau liquide/vapeur d'eau. Comme type d'appareil, on utilisera plus particulièrement celui vendu sous le nom ®MICROMIST par la Société TAKARA BELMONT.

Les procédés précédemment cités peuvent être mis en oeuvre le jour même de la coloration ou un jour ou plusieurs jours (de 1 à 60 jours) après la coloration.

L'invention a également pour objet un procédé de traitement des fibres kératiniques pour uniformiser la couleur desdites fibres suite à leur coloration. Préalablement à une étape de coloration capillaire, les fibres sont lavées, puis essorées, la composition cosmétique telle que définie ci-dessus et contenant un agent nucléophile est appliquée sur lesdites fibres.

Selon une variante du procédé défini ci-dessus, le procédé de traitement des fibres kératiniques pour uniformiser la couleur desdites fibres suite à leur coloration est réalisé en deux temps. Préalablement à une étape de coloration capillaire, les fibres sont lavées, puis essorées, une composition contenant un agent nucléophile est appliquée sur lesdites fibres. Les fibres sont ensuite éventuellement séchées. Puis dans un second temps, une composition cosmétique telle que définie ci-dessus et ne contenant pas d'agent nucléophile est appliquée sur lesdites fibres.

Il est possible de prévoir, préalablement au dépôt de la composition contenant l'agent nucléophile, une composition contenant un additif cosmétique est appliquée sur lesdites fibres.

Les procédés selon l'invention peut comprendre une étape supplémentaire de séchage total ou partiel des fibres kératiniques avec un séchoir et éventuellement un shampooing terminal.

Un autre objet de l'invention concerne un procédé de coloration consistant à appliquer sur les fibres kératiniques humaines et plus particulièrement des cheveux, une composition (A) colorante, cette composition pouvant contenir au moins un colorant direct (A1), ou au moins un colorant direct et un agent oxydant, conduisant à une composition de coloration directe éclaircissante (A2), ou au moins une base de coloration d'oxydation et éventuellement au moins un coupleur (A3), ou encore au moins une base de coloration d'oxydation et éventuellement au moins un coupleur, et au moins un colorant direct (A4), pendant un temps suffisant pour développer la couleur, et de faire suivre ou précéder cette application par l'application d'une composition (B) contenant la composition cosmétique telle que définie précédemment comprenant le monomère cyanoacrylate polymérisable et au moins un agent de conditionnement et/ou au moins un composé additionnel choisi parmi les charges, les bases minérales ou organiques et les alcools inférieurs en C₁-C₈, en présence d'au moins un agent nucléophile tel que défini précédemment ; ledit agent nucléophile étant présent dans ladite composition ou conditionné séparément dans une composition (C).

L'application de la composition colorante (A) peut être suivie d'un rinçage et/ou d'un séchage des fibres kératiniques.

L'application de la composition (B) peut être suivie d'un rinçage et/ou d'un séchage des fibres kératiniques. La composition (B) peut être préalablement chauffée dans les mêmes conditions définies ci-dessus. L'application de la composition (B) peut être suivie par un chauffage des fibres kératiniques dans les mêmes conditions définies ci-dessus.

La composition (B) peut être appliquée immédiatement après coloration ou de manière différée. Par différée, on entend une application se faisant quelques heures, un jour ou plusieurs jours (de 1 à 60 jours) après la coloration. De préférence, la composition (B) sera appliquée immédiatement après coloration des fibres kératiniques ; les applications de ladite composition pouvant être répétées entre deux colorations.

La nature et la concentration des colorants présents dans la composition (A) colorante n'est pas critique.

Par coloration au sens de la présente invention, on entend tout procédé de coloration qui met en oeuvre des colorants autres que les pigments, que l'on définit par toute entité organique et/ou minérale dont la solubilité dans l'eau est inférieure à 0,01 % à 20 °C, de préférence inférieure à 0,0001 %, et présentant une absorption entre 350 et 700 nm, de préférence une absorption avec un maximum.

Dans le cas des colorations directes éclaircissantes, les compositions colorantes (A2) résultent du mélange au moment de l'emploi d'une composition colorante contenant au moins un colorant direct et d'une composition (A5) contenant un agent oxydant.

Dans le cas des colorations d'oxydation, les compositions colorantes (A3) et (A4) résultent du mélange au moment de l'emploi d'une composition colorante contenant au moins une base d'oxydation et éventuellement au moins un coupleur et/ou au moins un colorant direct et d'une composition (C) contenant un agent oxydant.

Les colorants directs sont plus particulièrement des composés absorbant les radiations lumineuses dans le domaine visible (400-750 nm). Ils peuvent être de nature non ionique, anionique ou cationique.

Généralement, les colorants directs sont choisis parmi les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ceux dérivés du triarylméthane, seuls ou en mélanges.

Parmi les colorants benzéniques nitrés, on peut citer les composés rouges ou orangés suivants : le 1-hydroxy-3-nitro-4-N-(γ-hydroxypropyl)amino benzène, le N-(β-hydroxy éthyl)amino-3-nitro-4-amino benzène, le 1-amino-3-méthyl-4-N-(β-hydroxyéthyl)amino-6-nitro benzène, le 1-hydroxy-3-nitro-4-N-(β-hydroxyéthyl)-amino benzène, le 1,4-diamino-2-nitrobenzène, le 1-amino-2-nitro-4-méthylamino benzène, la N-(β-hydroxyéthyl)-2-nitro-paraphénylènediamine, le 1-amino-2-nitro-4-(β-hydroxy éthyl)amino-5-chloro benzène, la 2-nitro-4-amino-diphénylamine, le 1-amino-3-nitro-6-hydroxybenzène, le 1-(β-amino éthyl)amino-2-nitro-4-(β-hydroxy éthyloxy) benzène, le 1-(β, γ-dihydroxypropyl)oxy-3-nitro-4-(β-hydroxyéthyl)amino benzène, le 1-hydroxy-3-nitro-4-aminobenzène, le 1-hydroxy-2-amino-4,6-dinitrobenzène, le 1-méthoxy-3-nitro-4-(β-hydroxyéthyl)amino benzène, la 2-nitro-4'-hydroxydiphénylamine, le 1-amino-2-nitro-4-hydroxy-5-méthylbenzène, seuls ou en mélanges.

En ce qui concerne les colorants directs benzéniques nitrés, on peut mettre en oeuvre des colorants de ce type jaunes et jaune-verts, comme par exemple le 1-β-hydroxyéthyloxy-3-méthylamino-4-nitrobenzène, le 1-méthylamino-2-nitro-5-(β,γ-dihydroxypropyl)oxy benzène, le 1-(β-hydroxyéthyl)amino-2-méthoxy-4-nitrobenzène, le 1-(β-amino éthyl)amino-2-nitro-5-méthoxy-benzène, le 1,3-di(β-hydroxyéthyl)amino-4-nitro-6-chloro benzène, le 1-amino-2-nitro-6-méthyl-benzène, le 1-(β-hydroxyéthyl)amino-2-hydroxy-4-nitrobenzène, la N-(β-hydroxyéthyl)-2-nitro-4-trifluorométhylaniline, l'acide 4-(β-hydroxy éthyl)amino-3-nitro-benzènesulfonique, l'acide 4-éthylamino-3-nitro-benzoïque, le 4-(β-hydroxyéthyl)amino-3-nitro-chlorobenzène, le 4-(β-hydroxyéthyl)amino-3-nitro-méthyl benzène, le 4-(β,γ-dihydroxypropyl)amino-3-nitro-trifluorométhylbenzène, le 1-(β-uréido éthyl)amino-4-nitrobenzène, le 1,3-diamino-4-nitrobenzène, le 1-hydroxy-2-amino-5-nitrobenzène, le 1-amino-2-[tris(hydroxyméthyl)méthyl]amino-5-nitro-benzène, le 1-(β-hydroxyéthyl)amino-2-nitrobenzène, le 4-(β-hydroxyéthyl)amino-3-nitrobenzamide.

Il est de même envisageable d'utiliser des colorants benzéniques nitrés bleus ou violets, comme entre autres le 1-(β-hydroxyéthyl)amino-4-N,N-bis-(β-hydroxyéthyl)amino 2-nitrobenzène, le 1-(γ-hydroxypropyl)amino 4-N,N-bis-(β-hydroxyéthyl)amino 2-nitrobenzène, le 1-(β-hydroxyéthyl)amino 4-(N-méthyl, N-β-hydroxyéthyl)amino 2-nitrobenzène, le 1-(β-hydroxyéthyl)amino 4-(N-éthyl, N-β-hydroxyéthyl)amino 2-nitrobenzène, le 1-(β,γ-dihydroxypropyl)amino 4-(N-éthyl, N-β-hydroxyéthyl)amino 2-nitrobenzène, les 2-nitroparaphénylènediamines de formule suivante : 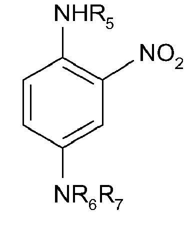 dans laquelle :
-R₆ représente un radical alkyle en C₁-C₄, un radical β-hydroxyéthyle ou β-hydroxypropyle ou γ-hydroxypropyle ;
R₅ et R7, identiques ou différents, représentent un radical β-hydroxyéthyle, β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans le brevet français FR 2 692 572.

Il est rappelé que les colorants azoïques sont des composés comportant dans leur structure au moins un enchaînement -N=N- non inclus dans un cycle ; les colorants méthiniques sont des composés comportant dans leur structure au moins un enchaînement -C=C- non inclus dans un cycle ; les colorants azométhinique sont des composés comportant dans leur structure au moins un enchaînement -C=N- non inclus dans un cycle.

Les colorants dérivés de triarylméthane comportent dans leur structure au moins un enchaînement suivant : 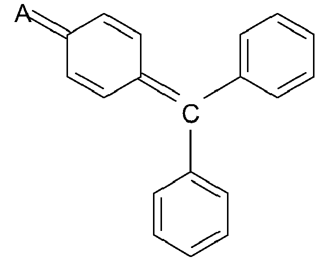 A désignant un atome d'oxygène ou d'azote

Les colorants xanthéniques comportent dans leur structure au moins un enchaînement de formule :

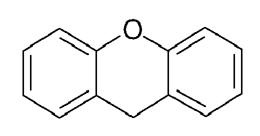

Les colorants phénanthridiniques comportent dans leur structure au moins un enchaînement de formule

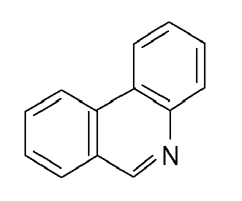

Les colorants phtalocyanines comportent dans leur structure au 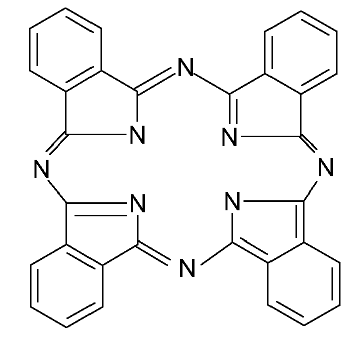 moins un enchaînement de formule :

Les colorants phénotiaziniques comportent dans leur structure au moins un enchaînement suivant :

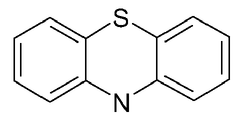

Les colorants directs peuvent de plus être choisis parmi les colorants basiques comme ceux listés dans le Color Index, 3ème édition, notamment sous les dénominations "Basic Brown 16", "Basic Brown 17", "Basic Yellow 57", "Basic Red 76", "Basic Violet 10", "Basic Blue 26" et "Basic Blue 99" ; ou parmi les colorants directs acides, listés le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore les colorants directs cationiques tels que ceux décrits dans les demandes de brevet WO 95/01772, WO 95/15144 et EP 714954 et en particulier « Basic Red 51 », « Basic Orange 31 », « Basic Yellow 87 » dont le contenu fait partie intégrante de la présente invention.

Les colorants directs peuvent également être choisis parmi les polymères colorés tels que décrits dans les demandes L'Oréal FR2361447, FR2456764, FR2457306, US3567678 et les demandes EP0747036, EP0852943, US4381260, US4533484, US4871371, US4911731, US4921589, US5310887, US5637637, US5951718, US6194534, US6342618, US6653390, US2002/0006977, US2003/0221587

Les colorants directs peuvent encore être choisis parmi les colorants directs hydrophobes dont le logP set supérieur à 2, tels que décrits dans les demandes FR 2 874 177 et FR 2 874 178, la valeur du logP représentant de façon classique le coefficient de partage du colorant entre l'octanol et l'eau.

Lorsqu'ils sont présents, le ou les colorants directs représentent de préférence de 0,0005 à 12 % en poids environ du poids total de la composition tinctoriale, et encore plus préférentiellement de 0,005 à 6 % en poids environ de ce poids.

Les bases d'oxydation peuvent être choisies parmi les bases d'oxydation classiquement utilisées en teinture d'oxydation et parmi lesquelles on peut notamment citer les paraphénylènediamines , les bis-phénylalkylènediamines, les paraaminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]-pyrimidin-7-yl)-(2-hydroxy-éthyl)-amino]-éthanol, le 2-[(7-amino-pyrazolo[1,5-a]-pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Lorsqu'elles sont utilisées, ces bases d'oxydation représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols, les naphtols et les coupleurs hétérocycliques tels que par exemple les dérivés indoliques, les dérivés indoliniques, les dérivés pyridiniques, les dérivés indazoliques, les dérivés de pyrazolo[1,5-b]-1,2,4-triazole, les dérivés de pyrazolo[3,2-c]-1,2,4-triazole, les dérivés de benzimidazole, les dérivés de benzothiazole, les dérivés de benzoxazole, les dérivés de 1,3-benzodioxole et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 6-hydroxybenzomorpholine, la 3,5 diamino 2,6-diméthoxy pyridine, le 1-N-(β hydroxyéthyl)amino 3,4 méthylènedioxy benzène, le 2,6 bis(β hydroxyéthylamino)toluène, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La nature de l'agent oxydant utilisé dans la coloration directe éclaircissante (coloration directe avec un agent oxydant) ou dans la coloration d'oxydation n'est pas critique.

L'agent oxydant est de préférence choisi dans le groupe formé par le peroxyde d'hydrogène, le peroxyde d'urée, les bromates ou ferricyanures de métaux alcalins, les persels tels que les perborates et les persulfates. On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Les compositions selon l'invention sont de viscosité variable. Elles peuvent être appliquées en gouttes successives puis malaxées au doigt, avec une éventuelle étape de peignage, ou appliquées au pinceau notamment en utilisant une planchette pour bien répartir la composition sur la longueur des fibres.

Dans le cas où la composition de l'invention est appliquée après une coloration, l'effet protecteur de la couleur du gainage conduit à une protection de la couleur plus homogène quand l'application est réalisée au pinceau. Ce mode d'application est donc une préférence.

L'invention a également pour objet un agent de coloration multi-composants ou kit comportant au moins un premier composant comprenant une composition (A1) de coloration directe telle que définie ci-dessus et un deuxième composant comprenant une composition (B) contenant la composition cosmétique contenant au moins un monomère cyanoacrylate polymérisable et au moins un agent de conditionnement et/ou au moins un composé additionnel choisi parmi les charges, les bases minérales ou organiques et les alcools inférieurs en C₁-C₈, telle que définie ci-dessus et éventuellement une composition (D) contenant au moins un agent nucléophile.

L'invention a également pour objet un agent de coloration multi-composants ou kit comportant au moins un premier composant comprenant une composition colorante, telle qu'une composition pouvant contenir au moins un colorant direct, ou une composition comprenant au moins une base de coloration d'oxydation et éventuellement au moins un coupleur (A3), ou encore une composition comprenant au moins une base de coloration d'oxydation et éventuellement au moins un coupleur, et au moins un colorant direct (A4), un deuxième composant comprenant une composition (B) contenant au moins un monomère cyanoacrylate polymérisable et au moins un agent de conditionnement et/ou au moins un composé additionnel choisi parmi les charges, les bases minérales ou organiques et les alcools inférieurs en C₁-C₈, telle que définie ci-dessus, un troisième composant comprenant une composition (C) comprenant au moins un agent oxydant ; et éventuellement un quatrième composant comprenant une composition (D) contenant au moins un agent nucléophile.

L'invention va être maintenant plus complètement illustrée à l'aide des exemples suivants qui ne sauraient être considérés comme la limitant.

### Eléments de démonstration

### Colorations testées

Sur mèches blanches de 1g naturels ou permanentés, nous avons réalisé des séries de mèches colorées à partir des produits commerciaux suivants :
- coloration d'oxydation :
   Majirouge 6.66
   Majirel 7.1
- coloration en mèches
   Maji Contrast nuance rouge cuivré
- coloration directe
   Color Pulse Rouge Pulse
   Renovative chatain cendré et châtain clair doré
   Expression nuance Cuivré
   Dédicace nuance Châtain auburn

### A. Tests de protection de la couleur et des propriétés cosmétiques après des lavages successifs

Deux mèches identiques sont réalisées pour chaque type de coloration.

Une mèche est shampooinée, une autre est traitée avec les compositions de l'invention puis reçoit le même nombre de shampooings.

La couleur des mèches est soumise à l'examen d'un panel d'experts. Des mesures colorimétriques sont également effectuées qui confirment les évaluations visuelles.

### Exemple 1 : coloration Maji Contrast Rouge Cuivré

La composition suivante est réalisée :

| | |
|---|---|
| poly diméthyl siloxane alpha-oméga dihydroxylé / cyclopentadiméthylsiloxane (14,7 / 85,3) commercialisé par Dow Corning sous le nom de DC 1501 Fluid | 45g |
| cyclopentadiméthylsiloxane commercialisé par Dow Corning sous le nom de DC245 Fluid | 44.75g |
| Acide acétique | 0.25 g |
| Méthylheptylcyanoacrylate de Chemence | 10g |

0.25g de cette composition est appliqué, de préférence au pinceau, sur une mèche de cheveux blancs de 1g préalablement colorée avec le Maji Contrast Rouge Cuivré. La mèche est séchée au casque pendant 30min puis peignée. Un shampooing terminal est éventuellement effectué. Les cheveux sont doux et déliés au toucher.

Après 15 shampooings la couleur de la mèche colorée puis traitée selon l'invention est nettement plus vive que la mèche colorée de façon identique par le Maji Contrast Rouge Cuivré et shampooinée 15 fois selon la même procédure.

### Exemple 2 : coloration Maji Contrast Rouge Cuivré

La composition suivante est réalisée :

| | |
|---|---|
| Huile d'olive | 90 g |
| Méthylheptylcyanoacrylate de Chemence | 10g |

0.25g de cette composition est appliqué, de préférence au pinceau, sur une mèche de cheveux blancs de 1g préalablement colorée avec le Maji contrast Rouge Cuivré. La mèche est séchée au casque pendant 30min puis peignée. Un shampooing terminal est éventuellement effectué. Les cheveux sont doux et déliés au toucher.

Après 15 shampooings la couleur de la mèche colorée puis traitée selon l'invention est nettement plus vive que la mèche colorée de façon identique par le Maji contrast Rouge Cuivré et shampooinée 15 fois selon la même procédure.

### Exemple 3 : coloration Maji Contrast Rouge Cuivré

La lotion **A** contenant 0.6 % de monoéthanolamine dans l'eau est réalisée.

La composition suivante **B** est réalisée :

| | |
|---|---|
| poly diméthyl siloxane alpha-oméga dihydroxylé / cyclopentadiméthylsiloxane (14,7 / 85,3) commercialisé par Dow Corning sous le nom de DC 1501 Fluid | 45g |
| cyclopentadiméthylsiloxane commercialisé par Dow Corning sous le nom de DC245 Fluid | 45 g |
| Méthylheptylcyanoacrylate de Chemence | 10g |

3 mèches de cheveux blancs de 1g, M1, M2 et M3 sont colorées par une coloration Maji contrast Rouge Cuivré.

0.25g de lotion **A** est appliqué sur la mèche **M1.** Puis 0.25g de la composition B est appliqué, de préférence au pinceau. La mèche est séchée au casque pendant 30min puis peignée. Un shampooing terminal est éventuellement effectué.

Parallèlement 0.25g de la composition B est appliqué, de préférence au pinceau, sur la mèche **M2**. La mèche est séchée au casque pendant 30min puis peignée. Un shampooing terminal est éventuellement effectué.

La mèche M1 est plus brillante que la mèche M2.

Après 15 shampooings la couleur des deux mèches colorées puis traitées selon l'invention M1 et M2 est nettement plus vive que la mèche M3 colorée de façon identique par le Maji contrast Rouge Cuivré et shampooinée 15 fois selon la même procédure.

L'association de la lotion A et de la composition B a permis d'améliorer l'aspect des mèches tout en conservant un effet protecteur de couleur de même niveau que celui obtenu avec la composition B seule.

### Exemple 4 : coloration Maji Contrast Rouge Cuivré

La composition suivante C est réalisée :

| | |
|---|---|
| poly diméthyl siloxane alpha-oméga dihydroxylé / cyclopentadiméthylsiloxane (14,7 / 85,3) commercialisé par Dow Corning sous le nom de DC 1501 Fluid | 45g |
| cyclopentadiméthylsiloxane commercialisé par Dow Corning sous le nom de DC245 Fluid | 41g |
| Nitrure de bore Boron nitride powder CC6004 de Advanced Ceramics | 4g |
| Méthylheptylcyanoacrylate de Chemence | 10g |

Selon les cas, on peut ajouter de l'acide acétique à hauteur de 0.25% au final dans la formule.

La composition suivante **D** est réalisée :

| | |
|---|---|
| poly diméthyl siloxane alpha-oméga dihydroxylé / cyclopentadiméthylsiloxane (14,7 / 85,3) commercialisé par Dow Corning sous le nom de DC 1501 Fluid | 45g |
| cyclopentadiméthylsiloxane commercialisé par Dow | 40g |
| Corning sous le nom de DC245 Fluid | |
| Silice hydrophobe Aerosil R8200 de Degussa | 5 g |
| Méthylheptylcyanoacrylate de Chemence | 10g |

Selon les cas, on peut ajouter de l'acide acétique à hauteur de 0.25% au final dans la formule.

La composition suivante **E** est réalisée :

| | |
|---|---|
| poly diméthyl siloxane alpha-oméga dihydroxylé / cyclopentadiméthylsiloxane (14,7 / 85,3) commercialisé par Dow Corning sous le nom de DC 1501 Fluid | 34g |
| cyclopentadiméthylsiloxane commercialisé par Dow Corning sous le nom de DC245 Fluid | 51.5g |
| Ethanol | 4.5 g |
| Méthylheptylcyanoacrylate de Chemence | 10g |

Selon les cas, on peut ajouter de l'acide acétique à hauteur de 0.25% au final dans la formule.

Cinq mèches blanches de 1g sont colorées par la coloration Maji contrast Rouge Cuivré.

Les mèches sont essorées, puis quatre mèches sont respectivement traitées par 0.25g de composition B, C, D et E appliquée de préférence au pinceau. Les mèches sont séchées au casque pendant 30 minutes puis peignées. La cinquième mèche est simplement séchée.

15 shampooings sont effectués sur les cinq mèches.

La couleur des mèches traitées par la composition B, C, D ou E est nettement plus vive que celle de la mèche simplement colorée et shampooinée.

### B. Tests pour comparer l'unisson d'une mèche colorée

La composition suivante est réalisée :

| | |
|---|---|
| poly diméthyl siloxane alpha-oméga dihydroxylé / cyclopentadiméthylsiloxane (14,7 / 85,3) commercialisé par Dow Corning sous le nom de DC 1501 Fluid | 45g |
| cyclopentadiméthylsiloxane commercialisé par Dow Corning sous le nom de DC245 Fluid | 44.75g |
| Acide acétique | 0.25 g |
| Méthylheptylcyanoacrylate de Chemence | 10g |

0.25g de cette composition est appliqué, de préférence au pinceau, sur une mèche de cheveux blancs de 1g permanentée.

La mèche est séchée au casque pendant 30min puis peignée. Un shampooing terminal est éventuellement effectué. Les cheveux sont doux et déliés au toucher.

Sur cette mèche M, on applique la coloration Maji Contrast Rouge cuivrée.

On applique également la coloration sur une mèche blanche permanentée témoin notée T.

On observe que la mèche M ayant reçu la composition contenant le cyanoacrylate a une couleur plus uniforme sur sa longueur que la mèche témoin T simplement colorée. On dit que la mèche M présente un meilleur unisson que la mèche témoin T.

## Revendications

1. Utilisation d'une composition cosmétique comprenant au moins un monomère cyanoacrylate polymérisable et au moins un agent de conditionnement et/ou au moins un composé additionnel choisi parmi les charges, les bases minérales ou organiques et les alcools inférieurs en C₁-C₈, pour améliorer la couleur de fibres kératiniques artificiellement teintes, notamment les fibres kératiniques humaines et plus particulièrement les cheveux humains.

2. Utilisation selon la revendication 1 pour protéger la couleur des fibres kératiniques et leurs propriétés cosmétiques après lavage.

3. Utilisation selon la revendication 1 pour améliorer l'unisson de la couleur des fibres kératiniques teintes artificiellement.

4. Utilisation selon l'une quelconque des revendications 1 à 3, **caractérisée en ce que** le monomère cyanoacrylate est de formule (I) : 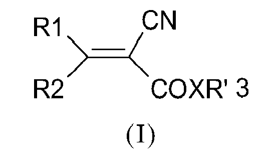 dans laquelle :
- X désigne NH, S ou O,
- R₁ et R₂ désignent chacun, indépendamment l'un de l'autre, un groupe peu ou non électro-attracteur (peu ou non inductif-attracteur) tel que :
- un atome d'hydrogène,
- un groupe hydrocarboné saturé ou non, linéaire, ramifié ou cyclique, comportant dé préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR, -COOR, -COR, -SH, -SR, -OH, et les atomes d'halogène,
- un résidu polyorganosiloxane modifié ou non,
- un groupement polyoxyalkylène,
- R désigne un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', - COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀.
- R'₃ représentant un atome d'hydrogène ou un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', -COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀.

5. Utilisation selon la revendication 4, **caractérisée en ce que** le monomère cyanoacrylate de formule (I) est choisi parmi les 2-cyanoacrylate d'alkyle ou d'alcoxyalkyle de formule (IV) : 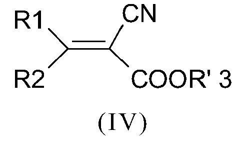 dans laquelle
R₁ et R₂ ont la même signification que pour la formule (I),
R'₃ représente un radical alkyle en C₁-C₁₀ ou alcoxy(C₁-C₄) alkyle(C₁-C₁₀).

6. Utilisation selon la revendication 5, **caractérisée en ce que** le monomère cyanoacrylate est choisi parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le 2-cyanoacrylate de 2-méthoxypropyle, le 2-cyanoacrylate d'allyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de 2-octyle, le 2-cyanoacrylate de 2-propoxyéthyle, le 2-cyanoacrylate de n-octyle et le cyanoacrylate d'iso-amyle.

7. Utilisation selon la revendication 5, **caractérisée en ce que** le monomère cyanoacrylate est choisi parmi les 2-cyanoacrylates d'alkyle en C₆-C₁₀.

8. Utilisation selon la revendication 7, **caractérisée en ce que** le monomère cyanoacrylate est choisi parmi les 2-cyanoacrylate d'octyle de formule (V) et leurs mélanges : 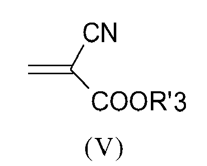 dans laquelle :
R'₃ =-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

9. Utilisation selon la revendication 8, **caractérisée en ce que** le monomère 2-cyanoacrylate est le méthylheptyl cyanoacrylate.

10. Utilisation selon l'une quelconque des revendications précédentes, **caractérisée en ce que** le monomère cyanoacrylate de formule (I) est fixé de façon covalente sur des supports tels que des polymères, des oligomères ou des dendrimères.

11. Utilisation selon l'une quelconque des revendications précédentes, **caractérisée en ce que** le monomère cyanoacrylate de formule (I) est présent dans une quantité allant de 0,1 à 50 % en poids, de préférence dans une quantité allant de 1 à 30 % en poids par rapport au poids total de la composition cosmétique.

12. Utilisation selon l'une quelconque des revendications précédentes, **caractérisée en ce que** l'agent de conditionnement est choisi parmi les acides organiques et les huiles.

13. Utilisation selon la revendication 12, **caractérisée en ce que** l'agent de conditionnement est un acide organique en C₁-C₁₂ portant un ou plusieurs groupements carboxyliques ou sulfoniques.

14. Utilisation selon la revendication 13, **caractérisée en ce que** l'acide organique est choisi parmi l'acide benzène- ou toluène-sulfonique, l'acide acétique, l'acide formique, l'acide propionique, l'acide benzoïque, les acides mono-, di- ou trichloroacétiques, l'acide salicylique et l'acide trifluoroacétique, l'acide citrique, l'acide octanoïque, l'acide heptanoïque et l'acide hexanoïque.

15. Utilisation selon la revendication 14, **caractérisée en ce que** l'acide organique est choisi parmi l'acide acétique, citrique ou octanoïque.

16. Utilisation selon l'une quelconque des revendications 13 à 15, **caractérisée en ce que** l'acide organique est introduit dans la composition entre 0,01% et 30% en poids par rapport au poids total de la composition ; plus préférentiellement entre 0,1% et 15% en poids par rapport au poids total de la composition.

17. Utilisation selon l'une quelconque des revendications 12 à 16, **caractérisée en ce que** l'agent de conditionnement est une huile minérale, organique ou végétale.

18. Utilisation selon la revendication 17, **caractérisée en ce que** l'huile est une huile végétale choisi parmi l'huile d'olive, l'huile de ricin, l'huile de colza, l'huile de coprah, l'huile de germes de blé, l'huile d'amandes douces, l'huile d'avocat, l'huile de polybutène, l'huile de macadamia, l'huile d'amandes d'abricot, l'isononate d'isononyle, l'huile de carthames, l'huile de noix de bancoulier, le malate d'iisostéaryle, l'huile de jojoba, l'huile de tournesol, l'huile de sésame, l'huile d'arachide, l'huile de pépins de raisin, l'huile de soja, e, l'huile de coprah, l'huile de maïs, l'huile de noisette, le beurre de karité, l'huile de palme, l'huile de calophyllum l'huile de camelina, l'huile d'amandes tamanu, le tetra-isostéarate de pentaerythrityle, le trimellitate de tridécyle, l'huile de citron.

19. Utilisation selon la revendication 18, **caractérisée en ce que** l'huile est l'huile d'olive.

20. Utilisation selon la revendication 17, **caractérisée en ce que** l'huile est une silicone volatile.

21. Utilisation selon la revendication 20, **caractérisée en ce que** l'huile est choisie parmi l'octaméthylcyclotétrasiloxane, le décaméthylcyclopentasiloxane, les cyclocopolymères du type diméthylsiloxane/méthylalkylsiloxane, les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane.

22. Utilisation selon la revendication 21, **caractérisée en ce que** l'huile est le décaméthylcyclopentasiloxane (ou D5).

23. Utilisation selon la revendication 17, **caractérisée en ce que** l'huile est une silicone fluide choisie parmi :
- les mélanges décaméthylcyclopentasiloxane et diméthiconol,
- les mélanges décaméthylcyclopentasiloxane et diméthicone,
- les mélanges PDMS fluide et diméthiconol.

24. Utilisation selon l'une quelconque des revendications 17 à 23, **caractérisée en ce que** l'huile est introduite dans la composition entre 1% et 99.9% en poids par rapport au poids total de la composition, plus préférentiellement entre 20% et 85% en poids par rapport au poids total de la composition.

25. Utilisation selon l'une quelconque des revendications précédentes, **caractérisée en ce que** le composé additionnel est une charge choisi parmi les charges minérales ou organiques.

26. Utilisation selon la revendication 25, **caractérisée en ce que** la charge est choisie parmi le talc, le mica, la silice, la silice pyrogénée éventuellement traitée en surface par un agent hydrophobe, le kaolin, la bentone, les poudres de polyamide, de poly-β-alanine et de polyéthylène, les poudres de polymères de tétrafluoroéthylène, la lauroyl-lysine, l'amidon, le nitrure de bore, les microsphères creuses polymériques telles que celles de chlorure de polyvinylidène/acrylonitrile, de copolymères d'acide acrylique et les microbilles de résine de silicone, les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

27. Utilisation selon la revendication 26, **caractérisée en ce que** la charge est de la silice pyrogénée éventuellement traitée en surface par un agent hydrophobe, de préférence par des groupements triméthylsiloxyle ou du nitrure de bore.

28. Utilisation selon l'une quelconque des revendications 25 à 27, **caractérisée en ce que** la charge est introduite dans la composition entre 0,01% et 50% en poids par rapport au poids total de la composition ; plus préférentiellement entre 0,01% et 30% en poids par rapport au poids total de la composition.

29. Utilisation selon l'une quelconque des revendications précédentes, **caractérisée en ce que** le composé additionnel est une base minérale ou organique choisie parmi l'ammoniaque, la monoéthanolamine, la diéthanolamine, la triéthanolamine, la 1,3-propanediamine, un carbonate ou un bicarbonate alcalin ou d'ammonium, un carbonate organique, un hydroxyde alcalin.

30. Utilisation selon la revendication 29, **caractérisée en ce que** la base est introduite dans la composition entre 0,01% et 30% en poids par rapport au poids total de la composition ; plus préférentiellement entre 0,01% et 5% en poids par rapport au poids total de la composition.

31. Utilisation selon l'une quelconque des revendications précédentes, **caractérisée en ce que** le composé additionnel est un alcool choisi parmi les monoalcools inférieurs ayant de 1 à 5 atomes de carbone ou les glycols ayant de 2 à 8 atomes de carbone.

32. Utilisation selon la revendication 31, **caractérisée en ce que** l'alcool est l'éthanol.

33. Utilisation selon la revendication 31 ou 32, **caractérisée en ce que** l'alcool est introduit dans la composition entre 0,01% et 30% en poids par rapport au poids total de la composition ; plus préférentiellement entre 0,01% et 5% en poids par rapport au poids total de la composition.

34. Utilisation selon l'une quelconque des revendications précédentes, **caractérisée en ce que** la composition comprend au moins un inhibiteur de polymérisation.

35. Utilisation selon la revendication 34, **caractérisée en ce que** la concentration en inhibiteur de polymérisation est comprise entre 10 ppm et 5%, et plus préférentiellement entre 10 ppm et 0,5% en poids par rapport au poids total de la composition.

36. Utilisation selon l'une quelconque des revendications précédentes, **caractérisée en ce que** la composition comprend au moins un filtre UV organique.

37. Utilisation selon l'une quelconque des revendications précédentes, **caractérisée en ce que** la composition comprend au moins un polymère.

38. Utilisation selon l'une quelconque des revendications précédentes, **caractérisée en ce que** la composition se trouve dans un support anhydre.

39. Utilisation selon l'une quelconque des revendications précédentes, **caractérisée en ce que** la composition comprend un support comprenant un ingrédient choisi parmi les alcools aromatiques, les alcools gras liquides comportant au moins 12 atomes de carbone, les cires oxyéthylénées ou non, les paraffines, les alcanes et plus particulièrement les alcanes de C₅ à C₁₀, les acides gras comportant au moins 12 atomes de carbone, les amides grasses, les esters gras et plus particulièrement les benzoates ou les salicylates d'alcool gras.

40. Utilisation selon l'une quelconque des revendications précédentes, **caractérisée en ce que** la composition cosmétique contient des additifs cosmétiques usuels choisis parmi les agents réducteurs, les agents oxydants, les séquestrants, les agents épaississants polymériques ou non, les agents hydratants, les agents émollients, les plastifiants, les azurants optiques, les argiles, les minéraux colloïdaux, les métaux colloïdaux, les particules de semi-conducteurs de type «puits quantiques» à base de métaux ou de silicium, un composé photo ou thermochromique, les agents nacrant, les parfums, les peptisants, les conservateurs, les protéines, les vitamines, les agents antipelliculaires, les cires oxyéthylénées ou non, les polymères anioniques, cationiques ou amphotères fixants ou non, les tensioactifs anioniques, cationiques, amphotères et non-ioniques.

41. Utilisation selon l'une quelconque des revendications précédentes, **caractérisée en ce que** la composition comprend au moins un agent nucléophile ; ledit agent nucléophile étant en mélange dans la composition telle que définie selon l'une quelconque des revendications précédentes ou conditionné séparément.

42. Utilisation selon la revendication 41, **caractérisée en ce que** l'agent nucléophile est l'eau.

43. Procédé pour améliorer la couleur des fibres kératiniques teintes artificiellement, notamment les fibres kératiniques humaines et plus particulièrement les cheveux humains, **caractérisé par le fait que** l'on applique avant ou après coloration desdites fibres, au moins une composition cosmétique telle que définie dans la revendications 1 à 40 en présence d'au moins un agent nucléophile selon la revendication 41 ou 42 ; ledit agent nucléophile étant mélangé au moment de l'emploi dans ladite composition ou appliqué séparément.

44. Procédé de coloration consistant à appliquer sur les fibres kératiniques teintes artificiellement, notamment les fibres kératiniques humaines et plus particulièrement les cheveux humains, une composition (A) colorante, cette composition pouvant contenir au moins un colorant direct (A1), ou au moins un colorant direct et un agent oxydant, conduisant à une composition de coloration directe éclaircissante (A2), ou au moins une base de coloration d'oxydation et éventuellement au moins un coupleur (A3), ou encore au moins une base de coloration d'oxydation et éventuellement au moins un coupleur, et au moins un colorant direct (A4), pendant un temps suffisant pour développer la couleur, et de faire suivre ou précéder cette application par l'application d'une composition (B) contenant la composition cosmétique telle que définie à l'une quelconque des revendications 1 à 40 en présence d'au moins un agent nucléophile selon la revendication 41 ou 42 ; ledit agent nucléophile étant mélangé au moment de l'emploi dans ladite composition ou appliqué séparément.

45. Agent de coloration multi-composants ou kit comportant au moins un premier composant comprenant une composition (A) colorante telle que définie à la revendication précédente, un deuxième composant comprenant une composition (B) contenant la composition cosmétique telle que définie à l'une quelconque des revendications 1 à 40, éventuellement un troisième composant comprenant une composition (C) comprenant au moins un agent oxydant et éventuellement un quatrième composant comprenant une composition (D) contenant au moins un agent nucléophile.
